# EUROPEAN PATENT APPLICATION

(11) **EP 3 081 636 A1**
(43) Date of publication of application: **19.10.2016**
(21) Application number: 15163676.8
(22) Date of filing: 15.04.2015
(51) Int. Cl.: C12N 1/20, C12R 1/225

(54) **USE OF LACTIC ACID BACTERIA FOR BIOPROTECTION**

(71) Applicant: University of Copenhagen, 1165 Copenhagen K (DK)
(72) Inventor: Skovgaard Vegge, Christina, 2100 Copenhagen Ø (DK); Halberg Larsen, Marianne, 2830 Virum (DK); Ovsepian, Armen, 2000 Frederiksberg (DK)
(74) Representative: COPA Copenhagen Patents

(57) **Abstract**

The present invention shows for the first time that lactic acid bacteria and/or their produced antimicrobial compounds can significantly reduce the survival of C. *jejuni* at low temperatures. *Lactobacillus salivarius* VH02 and *Lactobacillus salivarius* VHO7, isolated from chicken caeca, produce anti-*Campylobacter* compound(s), which can efficiently reduce or kill C. *jejuni* under cool conditions, as well as under optimal growth conditions. The results encourage the possibility of exploring these compounds in a chicken meat bio-reservative context.

## Description

### BACKGROUND OF THE INVENTION

According to a 2011 Centre for Disease Control and Prevention (CDC) report, approximately 48 million Americans get sick, 128,000 are hospitalized and 3,000 die each year from infections with foodborne pathogens, also known as foodborne illness.

Bacteria are the source of many foodborne diseases, usually due to improper food handling. Some bacteria, in small amounts, are not harmful to most healthy adults because the human body is equipped to fight them off. However, the trouble begins when pathogenic bacteria and other harmful pathogens multiply or when the infective dose is very small. This is often associated with improper cooking/cooling or cross contamination during preparation. Foods that are contaminated may not look, taste or smell any different from foods that are safe to eat. Symptoms of foodborne infections and poisoning vary from mild to severe to life threatening and can develop as quickly as within thirty minutes to a few hours or longer (up to several days) after intake of contaminated food.

In the last 20 years, *Campylobacter* has emerged as the most commonly reported cause of bacterial gastroenteritis in humans worldwide. Infections with *Campylobacter* typically result in acute illness with clinical symptoms ranging from mild to severe mucoid and bloody diarrhoea, and in rare cases reactive arthritis or the serious neurodegenerative Guillain Barré syndrome.

Poultry meat is considered to be the major source of sporadic Campylobacteriosis through cross-contamination to ready-to-eat foods and direct hand-to-mouth transfer during food preparation. *C. jejuni* is highly adapted to the avian gastrointestinal environment, and poultry flocks are frequently heavily colonized by *C. jejuni.*

During slaughter, *C. jejuni* is transferred to the poultry meat and the prevalence of *Campylobacter*-contaminated broiler carcasses has been estimated to 76% in EU (EFSA, 2011).

The disease burden of Campylobacteriosis and its sequelae is predicted to be 0.35 million disability adjusted life years (DALYs) per year in EU and the cost of Campylobacteriosis to public health and to lost productivity in the EU is estimated by the European Food Safety Authority (EFSA) to be around 2.4 billion EUR a year.

Several tools are used to control enteric pathogens in poultry. Competitive exclusions strategies and the use of specific probiotics and synbiotics have shown to be effective means, and recent research has been focused at inhibition of this pathogen by safe preservatives such as plant-derived compounds, bacteriophages, probiotics, antimicrobial peptides, and bacteriocins.

Lactic acid bacteria (LAB) produce a number of antimicrobial substances such as organic acids, ammonia, acetaldehyde, and bacteriocins with the capacity to inhibit growth of food spoilage and pathogenic organisms. Specifically, the bacteriocins are important antimicrobial peptides, which target either the cellular envelope or intracellular mechanisms of other microorganisms.

The focus of reducing *Campylobacter* in food production has primarily been to reduce the colonization of poultry. Several studies have therefore investigated the inhibitory effect of LAB strains and/or their antimicrobial components on *C. jejuni* under conditions mimicking the gastrointestinal environment.

The transfer of *Campylobacter* from the gastrointestinal content to the meat occurs together with the other microorganisms in the broiler gut flora. Due to the sensitive nature of *Campylobacter,* the survival of this organism outside the gastrointestinal environment is expected to be highly affected by these accompanying microorganisms.

*Campylobacter,* transferred to poultry meat during slaughter, are exposed to chilling and aerobic conditions before being packed and transmitted to the consumers. There is no reported data regarding effective bio preservative *anti-Campylobacter* effects of LAB strains or specifically bacteriocins against *Campylobacter* at lower temperatures and aerobic conditions or modified atmosphere mimicking the conditions of food processing and storage of poultry meat.

Such data would be important since the risk of infection with *Campylobacter* is strongly dependent on the bacterial load in food products (WHO, 2009), and a significant decreased risk of *Campylobacter*iosis can be obtained by a reducing of viable bacteria *Campylobacter.* Although not being able to proliferate, *C. jejuni* can survive for several weeks at 4° C, but the mechanisms promoting survival of *C. jejuni* in the cold remain poorly understood.

Thus, there is a need for the development of means that can prevent pathogens on food products in general and in particular at slaughterhouses.

### SUMMARY OF THE INVENTION

The present invention relates to two strains of Lactic acid bacteria (LAB) isolated from the caeca of Danish broilers and their use as bioprotection on food products.

The present inventors disclose herein that lactic acid bacteria (LAB) isolated from chickens were evaluated for their *anti-Campylobacter* effect. Two strains from chicken caeca faeces, identified as *Lactobacillus salivarius* (VHO2 and VHO7) by 16S rRNA sequencing, had strong inhibitory effect against *C. jejuni* NCTC1168 after co-cultivation in BHI broth at 37°C and 30°C under both microaerophilic and aerobic conditions.

In particular, at 37°C in microaerophilic atmosphere an approximately 7 log10-unit reduction of *C. jejuni* was observed compared to the control after 26 hours of incubation with L. salivarius VHO2. At 30°C microaerophilic and aerobically L. salivarius VHO2 caused approximate 1.5 and 3 log10-unit reductions of C. jejuni, respectively.

In its first aspect, the invention is directed to a *Lactobacillus* bacterium having all of the identifying characteristics of the bacterial strain of *Lactobacillus salivarius* deposited in the Deutsche Sammlung von Mikroorganismen und Zellkulturen GmBH (DSMZ) under the accession number DSM 32013 or a derivative thereof.

In its second aspect, the invention is directed to a *Lactobacillus* bacterium having all of the identifying characteristics of the bacteria strain of *Lactobacillus salivarius* deposited in the Deutsche Sammlung von Mikroorganismen und Zellkulturen GmBH (DSMZ) under the accession number DSM 32014 or a derivative thereof.

The *Lactobacillus salivarius* strains of the present invention inhibits food borne pathogens, such as but not limited to *Campylobacter,* and thus an important aspect of the present invention relates to strains of *Lactobacillus salivarius,* characterized in that said strain of *Lactobacillus salivarius* has the ability of inhibiting the growth of at least one food-borne pathogenic microorganism.

The invention further more relates to any composition of matter comprising a *Lactobacillus salivarius* strain deposited in the Deutsche Sammlung von Mikroorganismen und Zellkulturen GmBH (DSMZ) under the accession number DSM 32013 or a derivative thereof and/or DSM 32014 or a derivative thereof.

A further aspect of this invention is the supernatant(s) from a cell culture comprising a strain of *Lactobacillus salivarius* deposited in the Deutsche Sammlung von Mikroorganismen und Zellkulturen GmBH (DSMZ) under the accession number DSM 32013 or a derivative thereof and/or DSM 32014 or a derivative thereof. This aspect includes processes for preparing a *Lactobacillus salivarius* supernatant, said process comprising culturing cells of a strain of *Lactobacillus salivarius* according to the present invention, and separating the supernatant from the cultured cells, thereby obtaining said supernatant. This process also enables further isolation of antimicrobial compounds obtainable from the supernatant.

Another aspect relates to a cell suspension and/or lysate comprising the *Lactobacillus salivarius* strains of the present invention and a process of preparing said suspension by culturing the cells and optionally inactivating, lysing or killing the cultured cells thereby obtaining an antimicrobial cell suspension and/or lysate. Killing or death of bacteria is the irreversible loss of ability to reproduce.

Another important aspect of the present invention relates to the ability of inhibiting the growth of at least one pathogenic microorganism on food products at temperatures ranging from 2-30 °C, such as but not limited to temperatures between 2-15°C, because this temperature is typically used in the industrial preparation and storage of several food products.

An additional aspect relates to the ability to inhibit growth and survival of pathogenic bacteria independently of pH. Lactic acid bacteria's are known to produce organic acids thereby creating an acidic environment, which by itself can be limiting for survival and growth of pathogenic bacteria. The antimicrobial activity of the present invention is functional both under acidic and neutral conditions.

A further important feature is the capacity to inhibit growth and survival of pathogenic bacteria a both aerobic and microaerophilic conditions. Hereby associating the antimicrobial activity of the present invention to biopreservation in both food products processed under aerobic conditions and stored under aerobic or in modified atmosphere as well as in the gastrointestinal tract of livestock.

In a presently preferred embodiment, the present invention relates to a method for reducing the presence of *Campylobacter* on a food product, especially poultry, at a temperature ranging from 2-15 °C and aerobic or modified atmosphere conditions, said method comprising
a) providing a fresh [not frozen] food product at risk of *Campylobacter*,
b) adding to said food product a composition comprising a *Lactobacillus strain* capable of reducing the presence of *Campylobacter* on said fresh food product at temperatures ranging from 2-15 °C and under aerobic conditions,
c) storing said food product at temperatures ranging from 2-15 °C and under aerobic or microaerophilic or modified atmosphere conditions,
thereby reducing the presence of *Campylobacter* on said fresh food product.

### DETAILED DESCRIPTION OF THE INVENTION

In this study, the bio-protective capabilities illustrated by anti*-Campylobacter* effect of several LAB strains newly isolated from chicken feces were investigated under both microaerophilic and aerobic condition and at various temperatures. The most effective strains showed strong inhibitory effect (up to 7 log₁₀-unit reduction) *in vitro* (see example 4).

The present invention in its first aspect, relates to two specific LAB strains and their use as bio-protection on food products.

### Deposited LAB strains

### Lactobacillus salivarius VHO2

A culture sample of the microorganism was deposited on 11.03.2015 under the terms of the Budapest treaty at the Deutsche Sammlung von Mikroorganismen und Zellkulturen GmBH (DSMZ) under the accession number DSM 32013.

Thus, the present invention relates to a biologically pure *Lactobacillus salivarius* bacteria having all of the identifying characteristics of the bacteria deposited as DSM 32013.

In a presently preferred embodiment, the present invention relates to a strain of *Lactobacillus salivarius* deposited in the Deutsche Sammlung von Mikroorganismen und Zellkulturen GmBH (DSMZ) under the accession number DSM 32013 or a derivative thereof.

### Lactobacillus salivarius VHO7

A culture sample of the microorganism was deposited on 11.03.2015 under the terms of the Budapest treaty at the Deutsche Sammlung von Mikroorganismen und Zellkulturen GmBH (DSMZ) under the accession number DSM 32014.

Thus, the present invention relates to a biologically pure *Lactobacillus salivarius* bacteria having all of the identifying characteristics of the bacteria deposited as DSM 32014.

In a presently preferred embodiment, the present invention relates to a strain of *Lactobacillus salivarius* deposited in the Deutsche Sammlung von Mikroorganismen und Zellkulturen GmBH (DSMZ) under the accession number DSM 32014 or a derivative thereof.

### A derivative thereof

In the present context the term "a derivative thereof" relates to any modification or variation of the two strains caused by, but not limited to, e.g. mutation, plasmid transfer, prophage, recombination and/or epigenetics.

The scope of the present invention also encompasses the variations of the deposited strains of *Lactobacillus salivarius* obtained by mutation, variation or recombination, provided that the resulting strain has the ability of inhibiting the growth of food-borne pathogenic microorganisms.

### Culture

One embodiment of the present invention relates to a culture obtained from the strains of the present invention.

### Regarding Deposited Microbial Organisms [EXPERT SOLUTION, Rule 13 bis.6 (PCT)]

Furthermore, for all deposited microbial organisms mentioned in the present patent application, the following applies.

As regards the respective Patent Offices of the respective designated states, the applicants request that a sample of the deposited microorganisms stated above only be made available to an expert nominated by the requester until the date on which the patent is granted or the date on which the application has been refused or withdrawn or is deemed to be withdrawn.

In particular it is requested, that regarding:
EUROPE
   In respect to those designations in which a European Patent is sought a sample of the deposited microorganism will be made available until the publication of the mention of the grant of the European patent or until the date on which application has been refused or withdrawn or is deemed to be withdrawn, only by the issue of such a sample to an expert nominated by the person requesting the sample, and approved either i) by the Applicant and/or ii) by the European Patent Office, whichever applies (Rule 32 EPC).
CANADA
   The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.
AUSTRALIA
   The applicant hereby gives notice that the furnishing of a sample of a microorganism shall only be affected prior to the grant of a patent, or prior to the lapsing, refusal or withdrawal of the application, to a person who is a skilled addressee without an interest in the invention.
SINGAPORE
   The applicant hereby requests that the furnishing of a sample of a microorganism shall only be made available to an expert.

### Composition

In one aspect the present invention relates to a composition, comprising the *Lactobacillus salivarius* strain deposited in the Deutsche Sammlung von Mikroorganismen und Zellkulturen GmBH (DSMZ) under the accession number DSM 32013 or a derivative thereof and/or DSM 32014 or a derivative thereof.

A composition in the context of the present invention shall mean a blend or mixture of compounds.

The compositions of the present invention may be a composition, which comprises one or more physiologically acceptable carriers, excipients and/or diluents such as but not limited to bacterial media. The composition may, in addition, be one or more stabilizing compounds and/or one or more buffering compounds.

In one embodiment, the present invention relates to a composition comprising, as an effective component, the *Lactobacillus salivarius* strain deposited in the Deutsche Sammlung von Mikroorganismen und Zellkulturen GmBH (DSMZ) under the accession number DSM 32013 or a derivative thereof.

In one embodiment, the present invention relates to a composition comprising, as an effective component, the *Lactobacillus salivarius* strain deposited in the Deutsche Sammlung von Mikroorganismen und Zellkulturen GmBH (DSMZ) under the accession number DSM 32014 or a derivative thereof.

In a preferred embodiment, the composition comprises freeze-dried *Lactobacillus salivarius* stains as disclosed herein.

In another preferred embodiment, the composition comprises a lysate of the *Lactobacillus salivarius* stains as disclosed herein.

The present invention particularly relates to compositions comprising the strains according to the present invention for use in inhibiting the growth of food-borne pathogenic microorganisms.

### Supernatant

In different ecological niches such as the gastrointestinal tract, lactic acid bacteria (LAB) are known to produce a variety of antimicrobial compounds, and culture supernatant of *Lactobacillus* have been reported to exert antibacterial activity.

Thus, one aspect of the present invention relates to a supernatant from a cell culture comprising a strain of *Lactobacillus salivarius* deposited in the Deutsche Sammlung von Mikroorganismen und Zellkulturen GmBH (DSMZ) under the accession number DSM 32013 or a derivative thereof and/or DSM 32014 or a derivative thereof.

As understood by the skilled addressee a supernatant of the present invention encompasses both the supernatant from such cultures, and/or concentrates of such supernatant and/or fractions of such supernatant.

The term "supernatant" in the present context refers to a medium from a bacterial culture from which the bacteria have subsequently been removed, e.g. by centrifugation or filtration.

A supernatant of the present invention can readily be obtained by a simple process for preparing a *Lactobacillus salivarius* supernatant, said process comprising
a) culturing cells of a strain of *Lactobacillus salivarius* according to any of claims 1 and/or 2, and
b) potentially releasing of active compounds and/or extracellular components from VHO2 and VHO7 by various cellular treatments such as, but not limited to, acidic or alkaline modifications, sonication, detergents e.g. Sodium dodecyl sulfate (SDS) and/or Triton X, muralytic enzymes e.g. mutalolysin and/or lysozyme, salt and/or alcohol.
c) separating the supernatant from the cultured cells,
   thereby obtaining said supernatant.

In a particular preferred embodiment of this process, the supernatant composition is further subjected to a drying step to obtain a dried culture product.

The drying step may conveniently be freeze drying or spray drying, but any drying process which is suitable for drying of e.g. bacteriocins, also including vacuum drying and air drying, are contemplated.

Although the content of the supernatant produced by the *Lactobacillus* of the present invention is not yet characterized in details, it is known that certain *Lactobacillus* may produce bacteriocins that are small heat-stable proteins therefore the present inventors expect that even drying methods, which result in moderate heating of the culture eluate product, will result in active compositions.

### Lysate

A fluid containing the contents of lysed cells is called a lysate. A lysate contain active components of the bacterial cells and may be either crude, thus containing all cellular components, or partially and/or completely separated in separate fractions, such as extracellular components, intracellular components, proteins etc.

Numerous treatments of bacteria cells can cause lysis, such as, but not limited to: osmosis, phage infection, heating, sonication, pressure alterations, detergents, organic solvents, chemical and/or enzymatic treatments.

Thus, the present invention relates to a lysate of a *Lactobacillus salivarius* strain deposited in the Deutsche Sammlung von Mikroorganismen und Zellkulturen GmBH (DSMZ) under the accession number DSM 32013 or a derivative thereof and/or DSM 32014 or a derivative thereof.

### Cell suspension

The present invention may also in one embodiment be described as a cell suspension comprising the *Lactobacillus salivarius* strain deposited in the Deutsche Sammlung von Mikroorganismen und Zellkulturen GmBH (DSMZ) under the accession number DSM 32013 or a derivative thereof and/or DSM 32014 or a derivative thereof.

In the present context, the term cell suspension relates to a number of *Lactobacillus salivarius* bacteria according to the present invention dispersed or in suspension in a liquid e.g. a liquid nutrient medium, culture medium or saline solution.

The cells may be presented in the form of a cell suspension in a solution that is suitable for dispersion. The cell suspension can e.g. be dispersed via spraying, dipping, or any other application process.

The cells may be viable, but the suspension may also comprise inactivated or killed cells or a lysate hereof.

In one embodiment, the suspension of the present invention provides viable cells.

In another embodiment, the suspension of the present invention provides inactivated, killed or lysed cells.

The present inventors disclosed that the ultra violet (UV) radiation killed cells suspension maintained its *Campylobacter* inhibitory effect up to neutral pH adjustment, thus those skilled in the art would appreciate that the present invention also encompasses a process for preparing an antimicrobial cell suspension comprising
a) cultivating cells of a strain of *Lactobacillus salivarius* according to the present invention, and
b) killing and/or lysing the cultured cells
thereby obtaining a killed antimicrobial cell suspension.

The skilled addressee would recognise that there are many ways to kill a bacterium, but many of these methods such as e.g. antibiotics may not be feasible in a food related context. Thus, in a presently preferred embodiment, the cells are killed by UV radiation, heat, freezing, sonication, pressure alterations, alkaline/acidic conditions, chemical and/or enzymatic treatment.

### Antimicrobial compound

Bacteriocins are antimicrobial compounds produced by bacteria to inhibit other bacterial strains and species.

Lactic acid bacteria (LAB) are well known to produce bacteriocins and these compounds are of global interest to the food industry because they inhibit the growth of many spoilage and pathogenic bacteria, thus extending shelf life and safety of foods. Bacteriocins are typically considered to be narrow spectrum antibiotics. Moreover, bacteriocins of especially LAB display very low human toxicity and have been consumed in fermented food for millennia.

*Lactobacillus salivarius* is a well known probiotic bacterium frequently isolated from mammalian and avian gastrointestinal tracts, many of which produce bacteriocins of sub-classes IIa, IIb and IId. *Anti-Campylobacter* bacteriocins of L. salivarius have been investigated for their activity against *Campylobacter* colonization of chickens.

The present invention relates to an isolated antimicrobial compound obtainable from the strains deposited according to the present application. Such antimicrobial compound may for example be obtained from a supernatant or lysate resulting from the process described herein, further comprising an isolation step.

As it is illustrated herein in a non-restricted way, it has been found that the *Lactobacillus salivarius* strains of the present invention or compositions comprising said strains and/or the supernatant and/or lysate and/or a cell suspension are useful as an antimicrobial compound.

In the present context, the term antimicrobial compound relates to a compound that kills microorganisms, impair their survival or inhibits their growth.

Antimicrobial compounds can be grouped according to the microorganisms they act primarily against. For example, antibacterials are used against bacteria and antifungals are used against fungi. They can also be classified according to their function. Compounds that kill microbes are called microbicidal, while those that merely inhibit their growth are called microbiostatic.

In one embodiment, the present invention relates to an antimicrobial compound, which is microbicidal.

In one embodiment, the present invention relates to an antimicrobial compound, which is microbiostatic.

In one embodiment, the present invention relates to an antimicrobial compound, which is antibacterial.

In order to evaluate the potential bacteriocin production of VHO2 and VHO, their anti-*Campylobacter* activity were analyzed in agar block tests, thus examining the ability of these strains to produce antimicrobial compounds diffusing in the agar and hereby causing growth inhibition zones of *Campylobacter* (see example 5).

Significant growth inhibition of *C. jejuni* was observed around the blocks of L. salivarius VHO2 and VHO7, which indicates the potential production of anti-*Campylobacter* compounds by L. salivarius VHO2 and VHO7.

To further characterize the production of antimicrobial compounds by VHO2 and VHO7, their inhibitory activity on *C. jejuni* was investigated following various cellular treatments (see example 5).

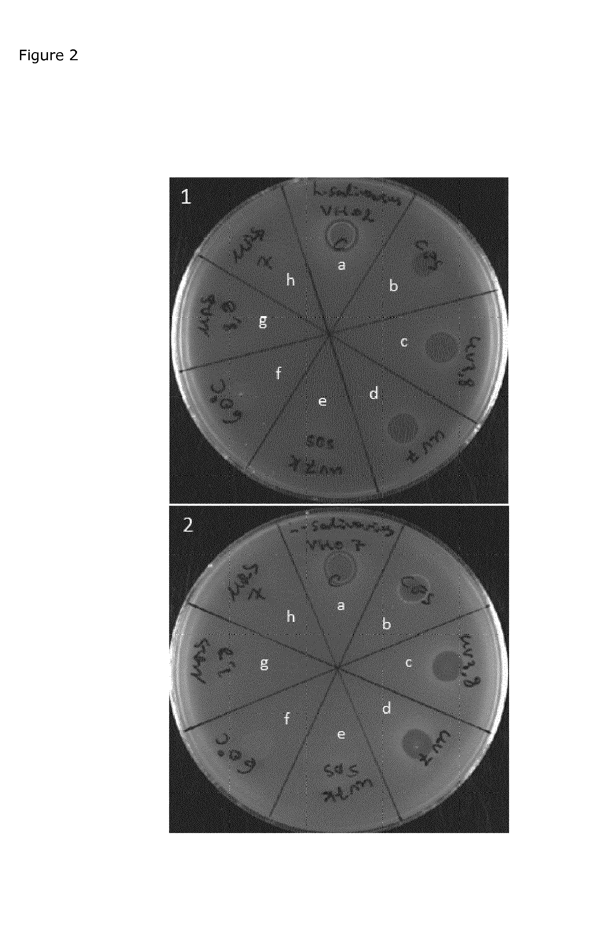

From these experiments, it was revealed that UV killed cells of VHO2 and VHO7 resuspended in saline buffer were able to inhibit growth of *C. jejuni* as efficiently as crude overnight cultures, and that this activity moreover was pH independent, as equally clear inhibition zones were observed with UV killed cells at both acidic and neutral pH (Fig. 2).

In contrast, the cell-free supernatants of VHO2 and VHO7 cultures were mainly active against *C. jejuni* at native acidic pH (Fig. 2).

This suggests that the putative antimicrobial compounds of VHO2 and VHO7 are associated with the cell envelope rather than being released to the supernatant. In support of this, the inhibitory activities of VHO2 and VHO7 were lost following treatment with Proteinase K in SDS and by SDS alone, but not significantly with proteinase K alone (Fig. 2 and results not shown). Most likely, the active component is masked by cellular components and therefore not accessible to proteinase K, while SDS dissolves the cellular envelope rendering the active compound susceptible to proteinase degradation as well as causing denaturation of the active compound. Additionally, the inhibitory activity of VHO2 and VHO7 was sensitive to heat treatment (60 °C, 30 min) (Example 5 and Fig. 2).

In summary, these results suggest *Lactobacillus salivarius* VHO2 and VHO7 to produce *anti-Campylobacter* compounds, which are heat labile, protease and SDS sensitive, functional at both acidic and neutral pH, and mainly associated with the cell envelope of the producing strains.

### Reducing pathogenic microorganisms

The LAB strains of the present invention were isolated from the caeca of Danish broilers using MRS agar to select for lactic acid bacteria (LAB). Individual isolates were investigated for their ability to inhibit *C. jejuni* upon co-cultivation *in vitro.*

*C. jejuni* NCTC11168 Δtlp1CamR and chicken caeca isolates were co-inoculated to an optical density (OD600) of 0.002 and incubated under either aerobic or microaerophilic conditions at various temperatures.

The ability of the caeca isolates to reduce *C. jejuni* CFU/ml (Colony Forming Units/ml) was followed over time, in comparison to control cultures of *C. jejuni* alone. In addition, endpoint culture pH was measured.

In total 40 caeca LAB isolates were screened for *anti-Campylobacter* activity under various environmental condition (30-37 °C, microaerophilic and aerobic atmosphere) (See Example 2 and 4).

A significant inhibitory effect on *C. jejuni* was observed for six isolates, all subsequently identified as *Lactobacillus salivarius* by 16s rRNA sequencing (Table 1).

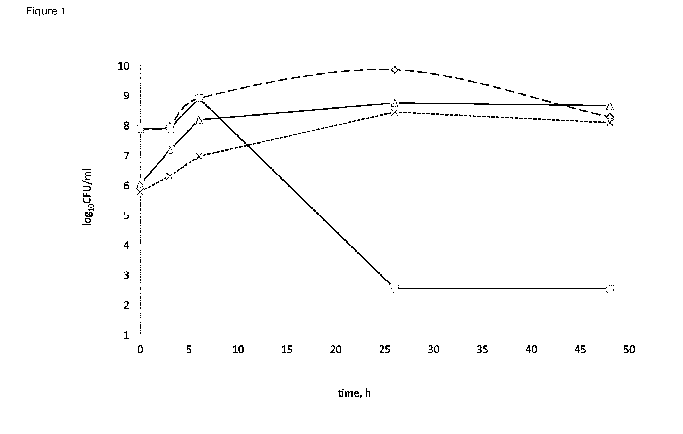

*Lactobacillus salivarius* VHO2 and VHO7 displayed the strongest anti*-Campylobacter* activity, as an approximately 7.5 log₁₀ reduction of *C. jejuni* NCTC11168 Δtlp1 CFU/ml was obtained upon 26 h co-cultivation with either of these strains at 37 °C in a microaerophilic atmosphere (see Fig. 1 and Table 2), while the remaining four *Lactobacillus salivarius* isolates (VHO3-6) displayed a 2.2-4.8 log10 reduction of *C. jejuni* CFU/ml under the same conditions (see Table 2).

At 30 °C in a microaerophilic atmosphere all *Lactobacillus salivarius* isolates caused a 2.0 log₁₀ reduction of *C. jejuni* NCTC11168 Δtlp1 CFU/ml following 48 h of co-cultivation, while a 2.4 log₁₀ reduction was obtained following 26 h at 30 °C under aerobic conditions (see Table 2).

### Reducing the undesired effects

In a presently preferred embodiment, the present invention relates to a *Lactobacillus salivarius* strain according to present invention, characterized in that said strain has the ability of reducing the undesired effects of at least one pathogenic microorganisms.

In the present context the term "reducing the undesired effects of pathogenic microorganisms" relates to any type of reduction of the undesired effects by these pathogenic microorganisms.

### Limiting, controlling or inhibiting the microorganisms survival and/or proliferation and/or ability to cause disease

In one embodiment, such reduction may be that the pathogenic microorganisms do not increase in numbers e.g. by simply just limiting, controlling or inhibiting the microorganisms proliferation and/or survival.

In another embodiment, such reduction may be that the pathogenic microorganisms in fact decrease in numbers. The decrease in numbers can be achieved by e.g. reducing the survival rate of the pathogenic microorganisms and/or by actual killing the pathogenic microorganisms.

In one embodiment, the undesired effect can be expressed in a CFU log₁₀ unit reduction.

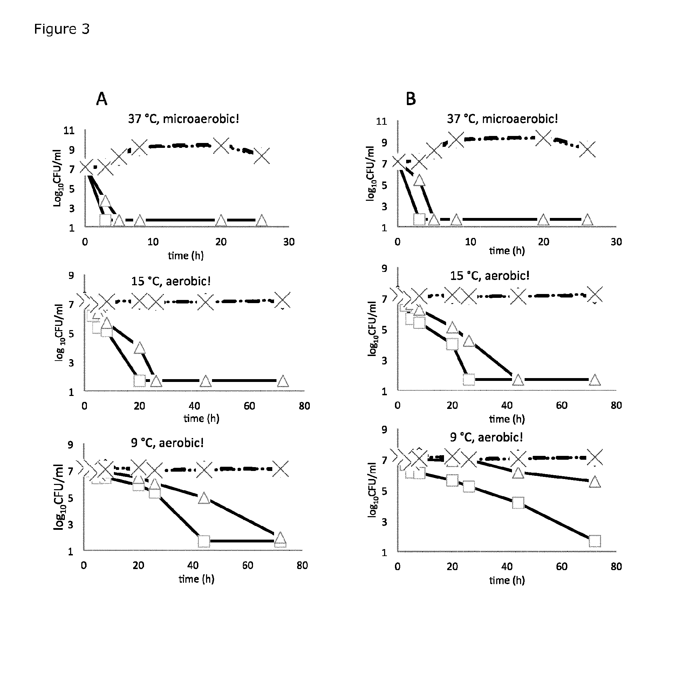

As disclosed in Table 2, Example 7 and Figure 3 of the present application, such CFU log₁₀ reduction may be e.g. 7.3 log₁₀ -unit.

The risk of infection is strongly dependent on the load of pathogenic bacteria in e.g. food products, and a significant decreased risk of infection can be obtained by reducing the load by e.g. a 1 log₁₀ reduction. Thus, in one embodiment the present invention relates to a CFU log₁₀ unit reduction of more than 1 CFU log₁₀ unit reduction, such as more than 1,2 CFU log₁₀ unit reduction, more than 1,5 CFU log₁₀ unit reduction, more than 1,75 CFU log₁₀ unit reduction, more than 2 CFU log₁₀ unit reduction, more than 2,5 CFU log₁₀ unit reduction, more than 3 CFU log₁₀ unit reduction, more than 3,5 CFU log₁₀ unit reduction, more than 4 CFU log₁₀ unit reduction, more than 4,5 CFU log₁₀ unit reduction, more than 5 CFU log₁₀ unit reduction, more than 6 CFU log₁₀ unit reduction, or more than 7 CFU log₁₀ unit reduction.

In another embodiment, such reduction may be weakening the pathogenic microorganism's ability to survive and/or proliferate upon transfer to another environment such as, but not limited to, other foods, food preparations, storage conditions and host organisms.

In one embodiment, the reduction of undesired effects of pathogenic microorganisms relates to the ability to reduce the virulence of pathogenic microorganisms, such as, but not limited to, reduced expression of colonization and virulence factors, and/or capacity to cause cellular adhesion and/or invasion.

Another embodiment relates to the ability to reduce the interaction of pathogenic bacteria and the host immune response, being both bacterial activation of the immune system and thereby causing disease as well as the ability of the bacteria to avoid the immune system and thereby being able to cause or exaggerate disease.

In one embodiment, the term "reducing the undesired effects" of microorganisms also encompass any type of bio-preservation against food-borne pathogenic microorganisms.

### Biopreservation of food products

As described herein, the present invention discloses biopreservation effects of the deposited *Lactobacillus salivarius* strains on food products by the same means as disclosed above.

This biopreservation effect may arise from e.g. the strains, cultures, supernatants, lysates, suspensions, compositions and/or antimicrobial compounds of the present invention.

In one embodiment, the present invention relates to the preservation of food products from undesired effects of food-borne pathogenic microorganisms by use of the strains, cultures, supernatants, lysates, suspensions, compositions and/or antimicrobial compounds of the present invention.

When evaluating the bio-protective effect of the present invention it may be important to determine the reduction of the cell counts compared to an unprotected culture. One such study could be as follows: Upon mixing and incubating pathogenic bacteria with VHO2 and VHO7, the cell counts of the pathogenic bacteria can be followed over time by plating appropriate 10-fold dilutions of the culture onto media specifically selecting for the pathogenic bacteria in question. Control cultures containing only pathogenic bacteria should be prepared in parallel. Evaluating the cell counts of the protected culture in comparison to the non-protected control culture provides a quantitative estimate of the bio-protective efficacy of VHO2 and VHO7 against the pathogenic bacterium.

### Spoilage

Spoilage is the process in which food deteriorates to the point in which it is not edible to humans or its quality of edibility becomes reduced. Various internal and external forces are responsible for the spoilage of food. Harvested foods decompose from the moment they are harvested due to attacks from enzymes, oxidation and microorganisms. The microorganisms include bacteria, mould, and yeast.

The present invention may also be useful for bio-protection of spoilage. The term "spoilage" as used herein refers to any type of process that acts to make damage or injuries that make the food undesirable for human consumption and unsaleable to consumers. Basic types of spoilage include changes in appearance, textural changes, and changes in taste or odour.

Bacteria, yeast and moulds can be responsible for the spoilage of food. When bacteria metabolize sugars and amino acids/proteins in the food, a complex mixture of acids, gases and other compounds such as volatile esters, alcohols, short fatty acids, diacetyl, ketones and sulphur-containing compounds are produced. While the bacteria themself may or may not be harmful, the bacterial mass and/or waste products may be unpleasant to taste, give an unpleasant odour, cause textural changes (slime formation), greening of meat and/or changes in appearance or may even be harmful to one's health. Yeasts can also be responsible for the decomposition of food by fermentation of the sugars and may occur in foods that have a high sugar content or high acid environment. The same effect is useful in the production of various types of fermented food and beverages, such as bread, yogurt, cider, and alcoholic beverages.

Examples of spoilage bacteria important in meat stored aerobic are *Pseudomonas, Acinetobacter, Psychrobacter, Moraxella, Brochothrix thermospacta, Enterobacteriaceae,* and lactic acid bacteria.

In vacuum-packed meat, lactic acid bacteria such as *Lactobacillus, Carnobacterium and Leuconostoc* and other bacteria such as psychrotrophic *Enterobacteriaceae,* dominate the spoilage microflora. *Shewanella* may also play a role in spoilage. In modified-atmosphere packed meat, *Pseudomonas* and *Brochothrix* can additionally be important.

In one embodiment, the present invention relates to delaying the appearance of unwanted sensory effects in food products such as, but not limited to, off-taste, off-odours or unwanted changes in appearance such as colour changes.

In one embodiment, the present invention relates to the delaying of food spoilage by bacteria.

In one embodiment, the present invention relates to inhibiting the growth and/or survival of food-borne pathogenic bacteria without causing sensory changes to the food.

### Pathogenic microorganisms

Typically, the term pathogenic microorganism is used to describe an infectious compound such as a virus, bacterium, prion, fungus, or parasite that causes disease in its host.

In a preferred embodiment, the term pathogenic microorganism relates to any microorganism having the ability to cause disease in a mammal. Reference to a "mammal" or a "subject" includes a human or non-human species including primates, livestock animals such as but not limited to chicken, duck, turkey, sheep, cows, pigs, horses, donkey, goats, laboratory test animals and companion animals.

In another preferred embodiment, the term pathogenic microorganism relates to any microorganism having the ability to cause disease in a human.

### Food-borne pathogenic microorganisms

Bacteria, parasites and viruses are the most common cause of food-borne infections and poisoning in humans. The symptoms and severity of the disease vary, depending on which bacteria, parasites or virus that cause the infection or food poisoning.

As used in a presently preferred embodiment, the term food-borne pathogenic microorganism refers to any food-borne microorganism that is capable of causing disease or illness in animals or humans.

The term food borne pathogenic microorganism would in a more preferably preferred embodiment include bacteria that are present in the food product and can causes disease or illness upon intake, as well as bacteria that produce toxins that cause poisoning or illness upon intake.

The bacteria, parasites and viruses that cause the most food related illnesses, hospitalizations, and deaths are:
*Salmonella*
*Staphylococcus aureus*
*Campylobacter*
*E. coli*
*Listeria monocytogenes*
*Clostridium*
*Yersinia*
*Arcobacter*
*Vibrio*
*Bacillus cereus*
*Shigella*
*Toxoplasma gondii*
*Norovirus* (Norwalk Virus)

Thus, in one embodiment a food-borne pathogenic microorganism relates to a microorganism selected from the group consisting of *Salmonella, Staphylococcus, Campylobacter, Escherichia, Listeria, Clostridium, Yersinia, Arcobacter, Vibrio, Bacillus, Shigella, Toxoplasma* and *Norovirus.*

Bacteria are the source of many cases of food poisoning and infections, thus in one embodiment, the food-borne pathogenic microorganism is a bacteria.

In another embodiment, a food-borne pathogenic microorganism relates to a microorganism selected from the group consisting of *Salmonella, Staphylococcus, Campylobacter, Escherichia, Listeria, Clostridium, Yersinia, Arcobacter, Vibrio, Bacillus,* and *Shigella.*

In one embodiment, the food-borne pathogenic microorganism relates to a microorganism selected from the group of Gram-negative bacteria consisting of *Salmonella, Campylobacter*, pathogenic *Escherichia, Yersinia, Shigella, Arcobacter* and *Vibrio.*

In one embodiment, the food-borne pathogenic microorganism relates to Gram-positive bacteria selected from the group consisting of *Staphylococcus, Listeria, Clostridium* and *Bacillus.*

In one embodiment, the pathogenic microorganism relates toxin-producing microorganism selected from the group consisting of *Staphylococcus, E. coli, Clostridium*, and *Bacillus.*

Helicobacter pylori, is a pathogenic bacterium found in the stomach of more than half of the world population. *H. pylori* is associated with chronic gastritis, gastric ulcers and the development of duodenal ulcers and stomach cancer. *H. pylori* is phylogenetically closely related to *Campylobacter* and these organisms share many physiological features.

Therefore, in one embodiment, the present invention relates to the ability of inhibiting *Helicobacter pylori.*

*Salmonella, Campylobacter, E. coli, Yersinia, Shigella* and *Vibrio* are zoonotic bacteria living in the intestines of livestock found on farms. These bacteria can be transmitted to humans after the subject has been slaughtered.

Therefore, in one embodiment, the present invention relates to the ability of inhibiting a zoonotic microorganism selected from the group consisting of *Salmonella, Campylobacter, E. coli, Yersinia, Shigella* and *Vibrio.*

*Campylobacter* and *Salmonella* bacteria live in the intestines of poultry and other livestock found on farms. These bacteria can be transmitted to humans after the subject has been slaughtered.

Thus, in a presently preferred embodiment, the present invention relates to the ability of inhibiting the growth of *Salmonella* and *Campylobacter.*

*Campylobacter* and *Arcobacter* are commensal bacteria of poultry but pathogenic in humans.

Therefore, in one embodiment, the present invention relates to the ability of inhibiting *Campylobacter* and *Arcobacter.*

*Campylobacter jejuni* is highly frequent commensal bacterium of poultry and other avian species. However, *C. jejuni* is pathogenic for humans.

*Campylobacter fetus* can cause abortion in sheep and cattle and moreover be pathogenic to humans.

In one embodiment, the term food-borne pathogenic microorganism as used herein refers to *Campylobacter,* preferably *Campylobacter jejuni and*/*or coli and*/*or fetus.*

In another embodiment, the term food-borne pathogenic microorganism as used herein refers to *Staphylococcus,* preferably *Staphylococcus aureus.*

In another embodiment, the term food-borne pathogenic microorganism as used herein refers to *Salmonella.*

In another embodiment, the term food-borne pathogenic microorganism as used herein refers to *E. coli.*

In the present context *E. coli* referes to diarrhoeagenic *Eschericha coli; Enteropathogenic E. coli, EnteroToxin producing E. coli, Enteroinvasive E. coli, Enteroaggregative E. coli, Verocytotoxin producing E. coli, Shiga toxin producing E. coli and Enterohamorrhagic E. coli.*

Among VTEC/STEC strains causing food borne infections, O157 is a very important serogroup because its effects can be extremely severe. Other important sero-groups are O26, O91, O103, O104, O111, and O145.

In another embodiment, the term food-borne pathogenic microorganism as used herein refers to *Listeria,* preferably *Listeria monocytogenes*.

In another embodiment, the term food-borne pathogenic microorganism as used herein refers to *Clostridium,* preferably *Clostridium perfringens* and/or *botulinum.*

In another embodiment, the term food-borne pathogenic microorganism as used herein refers to *Yersinia,* preferably *Yersinia enterocolitica*.

In another embodiment, the term food-borne pathogenic microorganism as used herein refers to *Arcobacter*, preferably *Arcobacter butzleri* and/or *cryaerophilus* and/or *skirrowii*.

*Vibrio* is typically found in aquatic environments and can cause foodborne infections, usually associated with eating undercooked seafood.

In another embodiment, the term food-borne pathogenic microorganism as used herein refers to *Vibrio,* preferably *Vibrio vulnificus* and/or *Vibrio parahaemolyticus* and/or *Vibrio cholera*.

In another embodiment, the term food-borne pathogenic microorganism as used herein refers to *Bacillus,* preferably *Bacillus cereus*.

In another embodiment, the term food-borne pathogenic microorganism as used herein refers to *Shigella*.

### Other Factors Affecting Microbial Growth

Many factors affect bacterial growth and survival and they are typically divided into environmental factors affecting growth, and sources of metabolic energy. The most important ones are:
Water - Bacteria need water to proliferate and prevent dormancy and/or sporulation. Water is required for all viable processed including transport of compounds across cellular membranes, enzymatic and chemical reactions, replication and growth.
2. Food/Nutrients - All bacteria require energy to proliferate. Energy sources such as sugars, starch, protein, amino acid and other compounds provide the nutrients.
3. Oxygen - Some bacteria require oxygen in order to grow (aerobes) while others can grow only in the absence of oxygen (anaerobes). However, many bacteria can grow under either condition and they are facultative anaerobes, while microaerophilic bacteria require reduced oxygen levels compared to atmospheric conditions.
4. Temperature - Bacteria in general are capable of growing over a wide range of temperatures and are usually classified according to the temperature interval at which they grow.
Psychrotrophic bacteria are capable of growing at 0°C - 7°C but their optimum is from 20°C to 30°C. They may cause spoilage of refrigerated foods. Some pathogenic bacteria are psychotrophic -- *Yersinia* and *Listeria*.
Mesophilic bacteria. Most bacteria are capable of growing at 15°C - 43°C and belong to this group. Most pathogenic bacteria grow at these temperatures. The optimal temperature for growth of most pathogenic bacteria is 37°C.
Thermophilic bacteria. These microorganisms can grow at higher temperatures such as 43°C - 65°C.
Temperature is the most widely used method of controlling bacterial growth. Bacteria grow slowly at temperatures below 7°C and thermal destruction occurs at temperatures above 60°C. But in the temperature danger zonebetween 4°C and 60°C -- many bacteria are not controlled.
5. pH - Most bacteria grow well at neutral pH, but many can reproduce in a pH range from 4.5 - 10.0. Most pathogenic bacteria grow best in pH 7.2-7.4

### Temperature

In order to explore the *anti-Campylobacter* activity of *L. salivarius* VHO2 and VHO7 under conditions of relevance for biopreservation, the reduction of *C. jejuni* was quantitated at cool temperatures. Killed suspensions of VHO2 or VHO7, pH 5.5/6.0 were mixed with *C. jejuni* and incubated at 37 °C in a microaerophilic atmosphere, and aerobically at 15 °C and 9 °C, respectively. The survival of *C. jejuni* was followed over time in comparison to control cultures without *Lactobacillus salivarius* cells.

This showed killed *L. salivarius* VHO2 to be able to significantly reduce *C. jejuni* to undetectable levels, corresponding to a 5 log10 reduction of CFU/ml under all investigated temperatures (see example 7 and Fig. 3).

Likewise, UV killed *L. salivarius* VHO7 UV were able to reduce *C. jejuni* following incubation at the equivalent conditions, but the anti-*Campylobacter* activity of VHO7 was a less efficient than VHO2 at the cooler temperatures (Fig. 3).

In contrast, no reduction or difference in *C. jejuni* survival was observed in the control cultures (Fig. 3).

A major cause of *Campylobacter* persisting as a food safety problem is the capacity of this organism to efficiently survive at cool temperatures during food storage. Therefore, the main advantage of VHO2 and VHO7 is that these strains are effective against *Campylobacter* at low temperature and under aerobic conditions. There are other studies showing *Campylobacter*-inhibitory effects of lactic acid bacteria (LAB), but only few reports on LABs effective at atmospheric oxygen concentrations and never before has efficient inhibitory activity against *Campylobacter* been shown at cold temperature.

The *Lactobacillus* of the present invention is capable of inhibition *C. jejuni* under both cold and aerobic conditions, thus in one embodiment the present invention relates to an antimicrobial compound according to the disclosure herein capable of inhibiting the growth and/or survival of at least one pathogenic microorganism on food products preserved at a temperature ranging from 2-30 °C.

The results disclosed herein show that the *anti-Campylobacter* compound produced by especially *Lactobacillus salivarius* VHO2 is indeed effective at reducing *C. jejuni* under conditions not supporting growth of this organism. Moreover, this reveals the potential of reducing the load of *C. jejuni* at cool temperatures, which is essential in the perspective of biopreservation.

In a preferred embodiment, the present invention relates to use of the *Lactobacillus* strains of the present invention in any of its formats e.g. characterized in that it has the ability of inhibiting the growth and survival of food-borne pathogenic microorganisms at non-optimum temperature for growth of common pathogenic microorganisms. Such temperature range may for example be between 2-20°C, 2-15°C, 2-10°C, 4-20°C, 4-15°C, 4-10°C, 6-20°C, 6-15°C, 6-10°C, 8-20°C, 8-15°C, 8-10°C, 10-30°C, 10-20°C, 10-15°C, 15-20°C, 20-25°C, 20-30°C, and/or 30-37°C.

Most food is stored or preserved in a refrigerated state at temperatures ranging from 2 to 10°C, thus since the present invention disclose that the inhibitory effect of the Lactobaccillus strains of the present invention is functional at 9°C, then a presently preferred embodiment of the present invention relates to an antimicrobial compound according to the disclosure herein capable of inhibiting the growth of at least one pathogenic microorganism on food products preserved in a refrigerated state at a temperature ranging from 2-10 °C

### Oxygen levels

A microaerophile is a microorganism that requires oxygen to proliferate, but requires environments containing lower levels of oxygen than are present in the atmosphere (i.e. <21% O₂; typically 2-10% O₂).

Many microaerophiles are also capnophiles, requiring an elevated concentration of carbon dioxide.

*Campylobacter* requires temperatures between 30-43 °C and a microaerophilic atmosphere and preferably elevated carbon dioxide to proliferate. Therefore *Campylobacter* can only grow under conditions being or mimicking the gastrointestinal environment, and there is no propagation of this bacterium in the environment or in food.

*Campylobacter* survives very poorly at warm temperatures in aerobic conditions, however it survives very efficiently at cool temperatures in aerobic conditions.

Most food products are processed, including slaughtering, under aerobic conditions, while storage of food occurs either aerobically or in modified atmosphere or in vacuum.

The *Lactobacillus* of the present invention is capable of inhibition of *C. jejuni* under both aerobic and microaerophilic conditions. Thus being able to control *Campylobacter* on food both under processing and storage.

It is envisioned that the *Lactobacillus salivarius* strains can be applied to the chicken carcases during slaughter thus targeting *Campylobacter* immediately at the time of contamination and continuing the activity throughout storage.

### pH

An overnight culture of *C. jejuni* NCTC11168 in BHI has a 7-7.2 pH value, while co-cultivation with the *Lactobacillus salivarius* strains reduced the endpoint pH to 4.8-5.2. We have previously shown that *C. jejuni* NCTC11168 is sensitive to acidic environments, displaying a 90-40% reduction in CFU/ml following 4 h incubation at pH 4.5-5 (Vegge et al., 2012). However, it is unlikely that the strong anti-*Campylobacter* activity of especially VHO2 and VHO7 is solely attributed to the pH reduction, as there were no correlation between reduction of *C. jejuni* CFU/ml and reduction of pH between six investigated *Lactobacillus salivarius* isolates (Table 2).

An additional aspect relates to the ability to inhibit growth and survival of pathogenic bacteria independently of pH. Lactic acid bacteria are known to produce organic acids thereby creating an acidic environment, which by it self can be limiting for survival and growth of pathogenic bacteria. The antimicrobial activity of the present invention is functional both under acidic (pH 3.8) and neutral (pH 7.0) conditions (Fig. 2).

The velocity of reduction *C. jejuni* by killed VHO2 and VHO7 was more efficient at pH 5.5 than 6.0, indicating the efficiency of the *anti-Campylobacter* compounds to be pH correlated but not dependent (Fig. 2 and 3).

### Reducing the load of Campylobacter

The reservoir for *Campylobacter* is the intestinal tract of animals, most commonly poultry. Although many sources of *Campylobacter* are recognized, Campylobacteriosis outbreaks are mainly associated with poultry meat. Research describing the prevalence of *Campylobacter* in retail poultry and by-products in various countries have found that the pathogen was present in 72% and 58% of samples in the US and Canada, respectively.

*Campylobacter* is a major food safety problem especially associated with poultry meat, since it is impossible to avoid transfer of *Campylobacter* from the intestinal content of broilers to the meat during industrial slaughter.

Poultry meat is often contaminated with feces during slaughter and is frequently identified as the source of human infection. Washing poultry carcasses with chemicals such as chlorine, organic acids, chlorine dioxide, trisodium phosphate or acidified sodium chlorite can reduce *Campylobacter* numbers by 0.5 to 1.5 log10 CFU/g. However, this is a negligible reduction compared to the high load of *Campylobacter* (5.0 to 8.0 log10 CFU/g of poultry feces) that frequently enter the processing area.

Therefore, application of natural antimicrobials to poultry products may contribute a much more acceptable alternative to significantly reduce the load of *Campylobacter* and consequently reduce the human cases of *Campylobacteriosis.*

The present inventors have isolated and discovered two strains of *Lactobacillus salivarius,* named VHO2 and VHO7, which can significantly reduce (up to 5 log₁₀) the survival of *Campylobacter* at cool temperatures, which makes VHO2 and VHO7 promising candidates for control of *Campylobacter* in food production.

Thus, one embodiment of the present invention relates to an antimicrobial compound wherein said compound is effective against *Campylobacter* at low temperature and under aerobic conditions and/or modified atmosphere and/or vacuum.

The effect can be obtained with both live and killed VH02 and VH07 cells, and it is envisioned that these *Lactobacillus salivarius* strains can be applied to e.g. chicken carcases during slaughter, thus targeting *Campylobacter* immediately at the time of contamination.

In one embodiment, the present invention relates to a method for reducing the load of Campylobacter on a food product at a temperature ranging from 2-15 °C and aerobic conditions, said method comprising
a) providing a fresh food product at risk of Campylobacter,
b) adding to said food product a *Lactobacillus salivarius* strain, culture, composition, supernatant, lysate, cell suspension and/or antimicrobial compound capable of reducing the undesired effects of Campylobacter on said fresh food product at temperatures ranging from 2-15 °C and under aerobic conditions,
c) storing said food product at temperatures ranging from 2-15 °C and under aerobic conditions,
thereby reducing the load of Campylobacter on said fresh food product.

In principle, the strains could be applied by aerosol spray to broiler carcasses during the evisceration (removal of internal organs), or applied by dipping the carcasses after evisceration thus inhibiting *Campylobacter* from the time of contamination.

### Food product

In the present application, the term food product may be any substance that can be used or prepared for use as food or any substance that can be metabolized by an human to give energy and build tissue, such as but not limited to meat, dairy products, vegetables, fruits and grains.

As used herein the term meat refers to any meat product or meat by-product including those processed from an animal which is consumed by humans or animal, such as but not limited to meat from bovine, ovine, porcine, poultry, fish and crustaceous seafood.

In the present application, the term food product is also intended to cover intermediary slaughter products.

### Fresh food products

In the present application, the term "fresh food product" is meant to cover food products that is not yet processed or manufactured, e.g. a fresh food product could be a meat product potentially contaminated by feces during slaughter, and/or it could be vegetables or other "ready to eat products", which include any food product, which does not require cooking prior to consumption.

In a presently preferred embodiment, said fresh food product is poultry.

In another presently preferred embodiment, said fresh food product is newly slaughtered poultry.

In the present context the term "poultry" relates to domesticated birds such as but not limited to members of the superorder Galloanserae (fowl), especially the order Galliformes (which includes chickens, quails and turkeys) and the family Anatidae, in order Anseriformes, commonly known as "waterfowl" and including domestic ducks and domestic geese. Poultry also includes other birds that are killed for their meat, such as the young of pigeons.

In particular preferred embodiment, said fresh food product is newly slaughtered chickens.

In one embodiment, the fresh food product is a ready to eat product (RTE), which is intended to include any food product, which does not require cooking prior to consumption. Such products may for example be slightly process meat and fish products such as, but not limited to, deli meat, smoked or gravid salmon or dairy products including, but no limited to, milk, yogurt, ice cream, cheese, butter cream and vegetables.

The present invention therefore can be described as treating fresh poultry meat with the *Lactobacillus* strains of the present invention or purified active compounds thereof to reduce the load of *Campylobacter.*

Production of the active *Lactobacillus salivarius* strains of the present invention is not costly and applying the strains as *anti-Campylobacter* compounds at the slaughterhouse should not require major changes of the processing line nor large equipment investments. The low cost is extremely important in the poultry industry where the profit per animal is quite small.

There are no efficient alternative technologies on the EU marked to reduce the load of *Campylobacter* on fresh poultry meat. In countries outside EU such as in USA and Canada chemical decontamination with substances such as chlorine dioxide, acidified sodium chlorite, trisodium phosphate and peroxyacids is used for poultry carcasses. In addition decontamination could use organic acids such as lactic acid and acetic acid or irradiation.

However, in EU besides using water to clean carcasses, decontamination treatments are not allowed to substitute good hygiene practices and can only be considered if a substance is shown to be safe and effective. To the best of our knowledge, chemical decontamination is currently not applied on poultry carcasses in the EU. Reductions obtained by hot water/steam have been shown to be in the range of 1-2 orders of magnitude but this treatment may exert an adverse impact on the carcass appearance. Moreover, hot water/steam has been tested to reduce the load of *Campylobacter* on poultry, but the method proved not to be very effective against this organism.

### Packaging in a modified atmosphere or in vacuum

Packaging fresh meat such as beef, pork and poultry in modified atmosphere packaging (MAP) or in vacuum will stall the growth of microbes that spoil fresh meat, and so both objectives, attractive appearance and a slow rate of spoilage, can be achieved. MAP and vacuum packaging are widely used in the food industry.

Using specific modified atmosphere packaging conditions, shelf life of red meat can typically be increased from around two to four days to between five and eight days under refrigeration, while that of poultry can be increased from 4-7 days to 16-21 days.

Thus, one embodiment of the present invention relates to the application of a strain of *Lactobacillus salivarius* or a derivative thereof and/or the antimicrobial compounds of the present invention prior to packaging in modified atmosphere.

Another embodiment of the present invention relates to the application of a strain of *Lactobacillus salivarius* or a derivative thereof and/or the antimicrobial compounds of the present invention prior to vacuum packaging.

Another embodiment of the present invention relates to coating packaging material with the *Lactobacillus* strains of the present invention and/or active compounds thereof in order to inhibit e.g. *Campylobacter* on the skin, meat and in meat juice.

One embodiment of the present invention relates to a method of reducing the undesired effects of at least one pathogenic microorganisms on a food product packed in a modified atmosphere or in vacuum characterised in that a strain of *Lactobacillus salivarius* or a derivative thereof, a culture, a composition, a supernatant, a lysate, a cell suspension and/or and antimicrobial compound according to the present application is applied to said food product prior to packaging.

### Feed additive

Upon consumption, probiotics deliver many lactic acid bacteria into the gastrointestinal tract. These microorganisms have been reputed to modify the intestinal milieu and to deliver enzymes and other beneficial substances into the intestines.

Research findings have revealed that prophylactic feeding of e.g. the poultry-specific, multi-species probiotic feed commercially known as PoultryStar to broilers causes a significant decrease in the cecal colonization of *Campylobacter jejuni* in trails with experimentally infected broilers.

Thus, in one embodiment, a strain, culture, composition, supernatant, lysate, cell suspension and/or antimicrobial compound according to the present invention can be used as a probiotic feed additive having beneficial effect on broiler performance, modulation of intestinal microflora and pathogen inhibition, intestinal histological changes, immunomodulation, certain haemato-biochemical parameters, improving sensory characteristics of dressed broiler meat and/or promoting microbiological meat quality of broilers.

### General

It should be understood that any feature and/or aspect discussed above in connections with the bacteria according to the invention apply by analogy to the methods and uses described herein.

The following figures and examples are provided below to illustrate the present invention. They are intended to be illustrative and are not to be construed as limiting in any way.

Those skilled in the art will appreciate that the invention described herein is adaptable to variations and modifications other than those specifically described. It is to be understood that the invention includes all such variation and modifications. The invention also includes all of the steps, features, compositions and compounds referred to or indicated in the specification, individually or collectively and any and all combinations or any two or more of the steps or features.

The present invention is not to be limited in scope by the specific embodiments described herein, which are intended for the purpose of exemplification only. Functionally equivalent products, compositions and where appropriate methods are clearly within the scope of the invention as described herein.

Throughout this specification and the claims that follow, unless the context requires otherwise, the word "comprise", or variations such as "comprises" or "comprising", will be understood to imply the inclusion of a stated integer or group of integers but not the exclusion of any other integer or group of integers.

### BRIEF DESCRIPTION OF THE FIGURES

*Figure 1* *- Reduction of C. jejuni by Lactobacillus salivarius VHO2 at optimal growth conditions.*
   *C. jejuni* NCTC11168 Δtlp1 cultivated in with and without *Lactobacillus salivarius* VHO2 in BHI at 37°C in a microaerophilic atmosphere. □, *C. jejuni* in co-culture, Δ,*Lactobacillus salivarius* VHO2 in co-culture, ◇, pure *C. jejuni* control culture, x, pure *Lactobacillus salivarius* VHO2 control culture. Results are representative of two independent experiments.
*Figure 2* *- UV killed Lactobacillus salivarius VHO2 and VHO7 inhibit growth of C. jejuni. Lactobacillus salivarius* VHO2 (1) and *Lactobacillus salivarius* VHO7 (2) subjected to various treatments spotted on *C. jejuni* NCTC11168 ΔTlp1 embedded in soft agar. a) Crude overnight culture of *Lactobacillus salivarius* VHO2/VHO7. b) Cell-free supernatant of *Lactobacillus salivarius* VHO2/VHO7 overnight culture. C) UV killed *Lactobacillus salivarius* VHO2/VHO7, pH 3.8 (corresponding to pH of overnight culture). d) UV killed *Lactobacillus salivarius* VHO2/VHO7, pH 7.0. e). UV killed *Lactobacillus salivarius* VHO2/VHO7, pH 7.0, treated with proteinase K (1mg/ml in 0.25% SDS). f) UV killed *Lactobacillus salivarius* VHO2/VHO7, pH 7.0, heat-treated (30 min, 60 °C). g) MRS medium, pH 3.8. h) MRS medium, pH 6.2 (native).
*Figure 3**- Reduction of C. jejuni at 37, 15 and 9 C by UV killed suspensions of L. salivarius VHO2 and VHO7*
   *C. jejuni* NCTC11168 Δtlp1 incubated in without and with *Lactobacillus salivarius* VHO2 (A) and *Lactobacillus salivarius* VHO7 (B). □, *C. jejuni* incubated with UV killed *L. salivarius* VHO2/VHO7, pH=5.5. Δ, *C. jejuni* incubated with UV killed *L. salivarius* VHO2/VHO7, pH=6.0. ◇, *C. jejuni* control, pH=5.5. x, *C. jejuni* control, pH=6.0.

### EXAMPLES

### Example 1 - Isolation of LAB and bacterial cultivation

Chicken intestines were collected in 2013 from Danish broilers during slaughter at a major slaughterhouse. For the isolation of Lactic acid bacteria (LAB), chicken caeca material was diluted in peptone saline diluent (Oxoid CM0733), spread on de Man-Rogosa-Sharp (MRS) agar (Oxoid CM0361) and incubated aerobically for 36-48 h at 37°C. Colonies of putative LAB were randomly picked and re-streaked twice on MRS agar and incubated aerobically for 36-48 h at 37°C. LAB isolates were stored in 17% glycerol at -80°C, streaked twice on MRS agar before each experiment and routinely cultivated in MRS broth.

*C. jejuni* NCTC11168 ΔTlp1 carrying a chromosomal chloramphenicol resistance marker was used throughout this study. Before each experiment, the freeze-stocked culture of *C. jejuni* was streaked twice on Base II (Oxoid CM0271) agar plates supplemented with 5% bovine blood and incubated at 37°C under microaerobic conditions (5% 02, 10% CO2, 85% N2) for 20-48 h. Liquid *C. jejuni* cultures were prepared in Brain Heart infusion (BHI) broth (Oxoid, CM1135), while selective enumeration from mixed cultures were carried out by plating serial dilutions on Base II agar supplemented with 20 µg/ml chloramphenicol.

### Example 2 - Initial screening of caecal isolates

40 individual bacterial isolates from the caeca content of broilers were screened for anti*-Campylobacter* activity. This was done in small scale co-cultivation assays and by agar well diffusion assays.

Co-cultivation screening: *C. jejuni* NCTC11168 Δtlp1CamR and individual chicken caeca isolates were co-inoculated to an optical density (OD600) of 0.002 in 3 ml BHI broth. Following 24 h of incubation at 37 °C under microaerophilic conditions (5% 02, 10% CO2, 85% N2), the levels of *C. jejuni* cells were enumerated by plating on Base II+5% blood agar containing chloramphenicol (20 µg/ml). Inhibitory activity against Campylobacter was evaluated in comparison to control cultures containing only C. jejuni, and thus it was revealed that 8 of the 40 tested LAB isolates displayed anti-Campylobacter activity (Table 1), with the activity of isolates 2-7 being stronger than isolates 1 and 8.

In addition, 30 µl of the overnight co-cultures were dispersed into 6 mm wells of agar plates seeded with a lawn of C. jejuni. Following overnight incubation at 37 C under micro aerophilic conditions, the ability of co-cultures to inhibit growth of C. jejuni around the wells was evaluated. Well-defined inhibition zones could not be seen in this assay, as co-cultures containing both LAB and *C. jejuni* were used to fill the wells, therefore only a qualitative score of anti-Campylobacter activity was observed for 8 of the 40 tested LAB isolates (Table 1).

### Example 3 - Species identification of VHO2 and VHO7

For species determination of LAB isolates, 16s rRNA regions were amplified by PCR using the primers: 8-27 forward (5'-AGAGTTTGATCCTGGCTAAG-3'), 1390-1408 reverse (5'-TGACGGGCGGTGTGTACAA-3'), 518-536 forward (5'-CCAGCAGCCGCGGTAATAC-3') 518-536 reverse (5'-GTATTACCGCGGCTGCTGG-3'), 785-805 forward (5'-GGATTAGATACCCAGGTAGTC-3') and 785-805 reverse (5'-GACTACCAGGGTATCTAATCC-3'). Amplified fragments were sequenced and by using the CLC Main Workbench 7.0.3 and BLAST VHO2, VHO3, VHO4, VHO5, VHO6 and VHO7 were determined to be isolates of *Lactobacillus salivarius.*

### Example 4 - Reducing C. jejuni by live Lactobacillus salivarius isolates

Co-cultures of *C. jejuni* and Lb salivarius were prepared by inoculating 12 ml BHI with 250 µl of exponentially growing (OD₆₀₀ 0.1) *C. jejuni* ΔTlp1 and 250 µl of diluted overnight culture (OD₆₀₀ 0.1) of individual LAB isolates. Control cultures containing only *C. jejuni* or LAB were prepared in parallel. The cultures were incubated under microaerophilic (5% O₂, 10% CO₂, 85% N₂) or aerobic conditions at 30 and 37°C. At various time intervals, diluted samples were spotted on MRS and Base II, 20 µg/ml Cam agar, for enumeration of LAB and *Campylobacter* cells respectively. The plating at each sampling point was done in duplicate and the result scored as the average CFU/ml. Thus the ability of the LAB isolates to reduce *C. jejuni* CFU/ml was followed over time in comparison to control cultures of C. jejuni. In addition, the culture pH was measured after 24-26h and 48h of incubation

A significant inhibitory effect on *C. jejuni* was observed for six *Lactobacillus salivarius* isolates (Table 2). Especially *Lactobacillus salivarius* VHO2 and VHO7 displayed the strongest *anti-Campylobacter* activity, as an approximately 7.3 log10 reduction of C. *jejuni* NCTC11168 Δtlp1 CFU/ml was obtained upon 26 h co-cultivation with either of these strains at 37°C in a microaerophilic atmosphere (Fig. 1, Table 2), while the remaining four *Lactobacillus salivarius* isolates (VHO3-6) displayed a 2.2-4.8 log10 reduction of *C. jejuni* CFU/ml under the same conditions (Table 2). At 30°C in a microaerophilic atmosphere all *Lactobacillus salivarius* isolates caused a 2.0 log10 reduction of *C. jejuni* NCTC11168 Δtlp1 CFU/ml following 48 h of co-cultivation, while a 2.4 log10 reduction was obtained following 26 h at 30°C under aerobic conditions (Table 2).

### Example 5 - Antimicrobial compound produced by LAB

In order to evaluate the potential bacteriocin production of VHO2, VHO7 and other LAB isolates, their *anti-Campylobacter* activity were analyzed in agar block tests. This was carried out according to Line et al., 2008 with some modifications. Briefly, approximately 10⁷ CFU from overnight cultures of LAB in MRS were spread on MRS agar and incubated aerobically at 37C. Agar blocks (diameter, 6 mm) containing individual LAB strains were aseptically excised from the agar plates and placed upside down on a lawn of *C. jejuni* embedded in soft agar. The soft agar lawns of *C. jejuni* were prepared by mixing 5ml Mueller-Hinton (MH) 0.7% soft agar (oxoid CM0405) with 200µl exponentially growing (OD₆₀₀ 0.1-0.2) *C. jejuni* NCTC11168 Δ*tlp1* and pouring onto solidified MH agar. Blocks of MRS agar without LAB were used as negative control, while 10 µl of 15% acetic acid was used as positive control. The plates containing *C. jejuni* and blocks were incubated under microaerophilic conditions for 24-36 h at 37°C. Diffusion of antimicrobial compounds from individual LAB isolates was evaluated by measuring the diameters of the inhibitory zones of *C. jejuni* growth from four replicates. The blocks of *Lactobacillus salivarius* VHO2 and VHO7 caused C. *jejuni* growth inhibition zones of 12.4 ± 0.3 and 12.2 ± 0.2 mm, respectively, while no inhibition could be observed for the other investigated isolates (results not shown). These results indicated the potential production of antimicrobial compounds by *Lactobacillus salivarius* VHO2 and VHO7.

The antimicrobial compounds produced by VHO2 and VHO7 were further characterized by spot tests. In this assay, 10 µl of VHO2 and VHO7 overnight cultures, cell-free supernatants as well as UV, heat, pH adjusted, SDS and proteinase K treated cell suspensions were spotted directly on a lawn of C. jejuni. Following overnight incubation at 37 C under microaerophilic conditions the resulting growth-inhibition of *C. jejuni* was evaluated as described above.

Cell free LAB supernatants were obtained by centrifugation (8000xg for 10min) and filtration (0.4 µm).

Killing of *Lactobacillus salivarius* by ultra-violet (UV) radiation was performed as described in Example 6.

Heat-treated samples were obtained by heating UV killed cell suspensions for 30 min at 60 C or 80°C.

Sodium dodecyl sulfate (SDS) and proteinase K treated samples were obtained by treating UV killed cell suspensions with 0.02% w/v SDS or 1 mg/ml proteinase K (Sigma Aldrich) in SDS (0.02% w/v) for 3 h at 37°C.

For the spot test, pH was adjusted with 100 mM NaOH or 100 mM HCl. MRS broth pH 6.2 (native media pH) and 3.8 (pH of *Lactobacillus salivarius* VHO2 overnight culture) were used as negative controls.

From these experiments, it was revealed that UV killed cells of VHO2 and VHO7 resuspended in saline buffer were able to inhibit growth of *C. jejuni* as efficiently as crude overnight cultures, and that this activity moreover was pH independent, as equally clear inhibition zones were observed with UV killed cells of pH 3.8 and 7.0 (Fig. 2).

In contrast, the cell-free supernatants of VHO2 and VHO7 cultures were mainly active against *C. jejuni* at pH 3.8 (native pH of supernatants), since only a minor effect was observed upon adjusting the supernatant pH to 4.5 (results not shown).

No inhibitory zones were observed in the control spots of pure MRS pH 3.8 and 6.2 (Fig. 2). Taken together these results suggest that the putative inhibitory compounds of VHO2 and VHO7 are associated with the cell envelope rather than being excreted to the supernatant. The inhibitory activities of VHO2 and VHO7 were lost following heat (60°C, 30 min) or SDS (0.02% w/v) treatment, but not significantly by proteinase K (1 µg/ml) alone without concomitant SDS (Fig. 2 and results not shown).

In summary, these results suggest *Lactobacillus salivarius* VHO2 and VHO7 to produce anti*-Campylobacter* compounds, which are heat labile, SDS sensitive, functional at neutral pH, and mainly associated with the cell envelope of the producing strains.

### Example 6 - Killing of Lactobacillus salivarius by ultra-violet (UV) radiation

Killing of *Lactobacillus salivarius* by ultra-violet (UV) radiation was performed as follows: 24 h cultures of *Lactobacillus salivarius* VHO2 and VHO7 in MRS (OD600 6.6 and 5.5, respectively) were harvested by centrifugation (8000xg for 10min) and resuspended in peptone saline diluent. Subsequently, the cell suspension was dispensed in 5 ml aliquots, killed by UV-treatment (0.1mJ/cm2 for 1-3 min), and restored to the original overnight cell density by centrifugation and resuspension with peptone saline diluent. The efficiency of the UV killing was verified by plating treated cell suspensions on MRS agar and incubating aerobically at 37°C for 48 h. No growth on plates was interpreted as efficient treatment.

### Example 7 - Reduction of C. jejuni survival under cold conditions with UV killed cell suspensions of Lactobacillus salivarius

The *anti-Campylobacter* activity of *L. salivarius* VHO2 and VHO7 under conditions of relevance for biopreservation, was explored by quantitating the reduction of *C. jejuni* at cool temperatures in comparison to the optimal growth conditions of C. jejuni.

UV killed cells solutions of *Lactobacillus salivarius* VHO2 or VHO7 were mixed in a 1:1 ratio with BHI, pH adjusted to 5.5 or 6.0 with 100mM HCl and inoculated with exponentially growing *C. jejuni* NCTC11168 Δtlp1 to OD600 0.002. Control cultures of *C. jejuni* without *Lactobacillus salivarius* cells, pH 5.5/6.0 were prepared in parallel. Cultures were incubated for up to 72 h at 15°C and 9°C under aerobic conditions, and at 37°C under microaerophilic conditions. The survival of *C. jejuni* was followed over time in comparison to control cultures by spotting diluted samples on Base II, 20 µg/ml Cam agar. The plating at each sampling point was done in duplicate and the result scored as the average CFU/ml.

This showed UV killed *L. salivarius* VHO2 to be able to efficiently reduce *C. jejuni* under growth-restrictive conditions, as *C. jejuni* was undetectable, corresponding to ca. 5.5 log₁₀ reduction of CFU/ml, following 20 and 44 h of incubation with VHO2 at 15°C and 9°C in pH 5.5 (Fig. 3). The efficiency of the effect was pH and temperature dependent, as the reduction time was a prolonged at pH 6.0, while significantly shorter at 37°C in a microaerophilic atmosphere. In contrast, no reduction of *C. jejuni* survival was observed in the pH 5.5/6.0 control cultures (Fig. 3). Likewise, UV killed L. *salivarius* VHO7 cells were able to reduce *C. jejuni* following incubation at the equivalent conditions, but the *anti-Campylobacter* activity of VHO7 was a less efficient than VHO2 at 15°C and 9°C (Fig. 3).

### Tables

**Table 1. Screen of 40 LAB caeca isolates for anti-Campylobacter activity.**

| **Isolate No** | **Co-cultultivation** | **Agar well diffusion** |
|---|---|---|
| 1 | + | + |
| 2 (*Lactobacillus salivarius* VHO2) | + | + |
| 3 (*Lactobacillus salivarius*) | + | + |
| 4 (*Lactobacillus salivarius*) | + | + |
| 5 (*Lactobacillus salivarius*) | + | + |
| *6* (*Lactobacillus salivarius*) | + | + |
| *7* (*Lactobacillus salivarius* VHO7) | + | + |
| 8 | + | + |
| 9 | - | - |
| 10 | - | - |
| 11 | - | - |
| 12 | - | - |
| 13 | - | - |
| 14 | - | - |
| 15 | - | - |
| 16 | - | - |
| 17 | - | - |
| 18 | - | - |
| 19 | - | - |
| 20 | - | - |
| 21 | - | - |
| 22 | - | - |
| 23 | - | - |
| 24 | - | - |
| 25 | - | - |
| 26 | - | - |
| 27 | - | - |
| 28 | - | - |
| 29 | - | - |
| 30 | - | - |
| 31 | - | - |
| 32 | - | - |
| 33 | - | - |
| 34 | - | - |
| 35 | - | - |
| 36 | - | - |
| 37 | - | - |
| 38 | - | - |
| 39 | - | - |
| 40 | - | - |

| | | |
|---|---|---|
| + indicates that inhibitory effect was detected. Individual LAB strains were isolated at different samplings of chicken caecum contents, and their order in the table does not represent the chronological order in which they are isolated. See example 2 for experimental details. | | |

**Tabel 2 - Inhibition of C. jejuni by LAB strains isolated from chicken caeca.**

| | Microaerobic, 37 °C | | Microaerobic, 30 °C | | | | Aerobic 30 °C | |
|---|---|---|---|---|---|---|---|---|
| Caecum fecal isolate | log₁₀ reduction in C. *jejuni* after 26 h^{a} | pH of co-culture^{b} | log₁₀ reduction in C. *jejuni* after 26 h^{a} | pH of co-culture after 26 h | log₁₀ reduction in C. *jejuni* after 48 h^{a} | pH of co-culture after 48 h | log₁₀ reduction in C. *jejuni* after 26 h^{a} | pH of co-culture^{b} |
| *Lactobacillus salivarius* VHO2 | 7.3 | 4.8 | 1.5 | 5.2 | 2.0 | 5.2 | 2.4 | 5.3 |
| *Lactobacillus salivarius* VHO3 | 2.2 | 5.2 | 0.0 | 5.2 | 2.0 | 5.2 | 2.4 | Nd^{c} |
| *Lactobacillus salivarius* VHO4 | 3.1 | 5.1 | 0.3 | 5.2 | 2.0 | 5.2 | 2.4 | Nd^{c} |
| *Lactobacillus salivarius* VHO5 | 2.4 | 5.0 | 0.4 | 5.2 | 2.0 | 5.3 | 2.4 | Nd^{c} |
| *Lactobacillus salivarius* VHO6 | 4.8 | 5.1 | 0.5 | 5.2 | 2.0 | 5.2 | 2.4 | Nd^{c} |
| *Lactobacillus salivarius* VHO7 | 7.3 | 5.0 | 0.1 | 5.2 | 2.0 | 5.3 | 2.4 | 5.3 |

| | | | | | | | | |
|---|---|---|---|---|---|---|---|---|
| ^{a}The log₁₀ reduction of *C. jejuni* NCTC11168 *ΔTlp1*CFU/ml is presented as the difference between *C. jejuni* CFU/ml in control and co-cultures. ^{b}Endpoint measurement ^{c}Not determined. | | | | | | | | |

## Claims

1. A strain of *Lactobacillus salivarius* deposited in the Deutsche Sammlung von Mikroorganismen und Zellkulturen GmBH (DSMZ) under the accession number DSM 32013 or a derivative thereof.

2. A strain of *Lactobacillus salivarius* deposited in the Deutsche Sammlung von Mikroorganismen und Zellkulturen GmBH (DSMZ) under the accession number DSM 32014 or a derivative thereof.

3. A culture comprising a *Lactobacillus salivarius* strain according to claim 1 and/or 2.

4. A supernatant from a cell culture according to claim 3.

5. A composition comprising a *Lactobacillus salivarius* strain according to claim 1 and/or 2.

6. A cell suspension or lysate prepare from a *Lactobacillus salivarius* strain according to claim 1 and/or 2.

7. A strain of *Lactobacillus salivarius* according to any of claims 1-2 and/or a culture according to claim 3 and/or a supernatant according to claim 4 and/or a composition according to claim 5 and/or a cell suspension or a lysate according to 6 for use as an antimicrobial compound.

8. An antimicrobial compound according to claim 7, for reducing the undesired effects of at least one pathogenic microorganism on food products.

9. An antimicrobial compound according to claim 8, capable of reducing the undesired effects of at least one pathogenic microorganism on food products preserved at a temperature ranging from 2-30 °C.

10. An antimicrobial compound according to claim 7-9, wherein said compound is effective against *Campylobacter* at low temperature and under aerobic conditions.

11. A method of reducing the undesired effects of at least one pathogenic microorganisms on a food product packed in a modified atmosphere or in vacuum **characterised in that** a strain of *Lactobacillus salivarius* or a derivative thereof, a culture, a composition, a supernatant, a lysate, a cell suspension and/or and antimicrobial compound according to any of the claims 1-10 is applied to said food product prior to packaging.

12. A feed additive comprising a strain of *Lactobacillus salivarius* or a derivative thereof, culture, composition, supernatant, lysate, cell suspension and/or antimicrobial compound according to any of the claims 1-10.

13. A method for reducing the load of *Campylobacter* on a food product at a temperature ranging from 2-15 °C and aerobic conditions, said method comprising
a) providing a fresh food product at risk of *Campylobacter,*
b) adding to said food product a *Lactobacillus salivarius* strain, culture, composition, supernatant, lysate, cell suspension and/or antimicrobial compound capable of reducing the undesired effects of *Campylobacter* on said fresh food product at temperatures ranging from 2-15 °C and under aerobic conditions,
c) storing said food product at temperatures ranging from 2-15 °C and under aerobic conditions,
thereby reducing the load of *Campylobacter* on said fresh food product.
